# EUROPÄISCHE PATENTSCHRIFT

(11) **EP 1 990 188 B1**
(45) Veröffentlichungstag und Bekanntmachung des Hinweises auf die Patenterteilung: **17.10.2012**
(21) Anmeldenummer: 08008612.7
(22) Anmeldetag: 07.05.2008
(51) Int. Cl.: B32B 37/14, B32B 37/10, A61F 13/15, B32B 27/12, B32B 37/15

(54) **Verfahren zur Herstellung eines Flächenmaterials sowie entsprechendes Flächenmaterial, Windelohr bzw. Windelverschlussband und Windel**
Method for producing a flat material and corresponding flat material, fastener and nappy close strap and nappy
Procédé de fabrication d'un matériau de surface et matériau de surface correspondant, bord de couche ou bande de fermeture de couche et couche

(30) Priorität: 08.05.2007 DE 102007022046
(43) Veröffentlichungstag der Anmeldung: 12.11.2008
(73) Patentinhaber: Koester GmbH & Co. KG, 96146 Altendorf (DE)
(72) Erfinder: Neugebauer, Robert, 91077 Neunkirchen (DE); Lanz, Jörg, 91052 Erlangen (DE); Schober, Uwe, 96179 Rattelsdorf (DE)
(74) Vertreter: Reuther, Martin

(56) Entgegenhaltungen:
- WO-A2-2008/005822
- DE-A1-102007 050 874
- US-B1- 6 313 372

## Beschreibung

Die Erfindung betrifft ein Verfahren zur Herstellung eines Flächenmaterials mit elastischen und nicht-elastischen Bereichen sowie mit einem textilen Charakter zumindest auf einer Oberflächenseite sowie ein entsprechendes Flächenmaterial. Ebenso betrifft die Erfindung ein Windelohr bzw. ein Windelverschlussband mit einem Fasteningträger aus einem entsprechenden Flächenmaterial sowie eine Windel mit einem derartigen Windelohr bzw. Windelverschlüssband.

Derartige Flächenmaterialien sind beispielsweise aus der WO 2001/53076 A1 aber auch aus der WO 2006/063232 A1 bzw. aus der EP 1 462 556 B1 und der WO 2006/071426 A1 bekannt.

Hierbei schlägt insbesondere die WO 2001/53076 A1 ein an sich eigensteifes NonWoven vor, welches bei einer Dehnung von etwas mehr als 100% bricht und partiell beidseits eines elastischen Films angeordnet und mit diesen partiell verbunden ist. Andererseits beschreiben die übrigen Druckschriften entsprechende Anordnungen aus NonWoven und elastischem Film, bei welchem elastische Bereiche durch Überstrecken des an sich nicht-elastischen Flächenmaterials elastifiziert werden.

Um diese Flächenmaterialien dahingehend auszugestalten, dass elastische Bereiche und nicht-elastische Bereiche vorliegen, werden bei der WO 2001/53076 A1 die Bereiche, die nicht-elastisch ausgebildet sein sollen, in einem separaten Verfahrensschritt versteift. Dieses kann beispielsweise dadurch geschehen, dass separate Streifen eingefügt, Klebstoff in das NonWoven eingebracht oder eine großflächige Verschmelzung zwischen NonWoven und elastischen Film vorgenommen wird. Die übrigen Druckschriften hingegen schlagen vor, die elastischen Bereiche durch Überstrecken in einem nachgeschalteten Verfahrensschritt zu aktivieren.

Einen hiervon abweichenden Weg schlägt die WO 2007/061486 A1 vor, nach welcher zunächst das NonWoven oder elastische Film in ausgewählten vorgestreckt und dann beide Lagen miteinander verbunden werden sollen. Hierdurch sollen in diesen Bereichen dem Flächenmaterial ausreichend Freiheit bereitgestellt werden, so dass dieses dann in diesen Bereichen elastisch verbleibt.

Darüber hinaus ist aus der US 6,313,372 Verfahren zur Herstellung eines Flächenmaterials bekannt, bei welchem das NonWoven und der elastische Film thermisch unter Nutzung verschiedener Temperaturen miteinander verbunden werden. Hierdurch wird dann ein Flächenmaterial mit elastischen und nicht-elastischen Bereichen sowie mit einem textilen Charakter zumindest auf einer Oberfläche bereitgestellt.

Es ist Aufgabe vorliegender Erfindung, ein gattungsgemäßes Verfahren zur Herstellung eines Flächenmaterials sowie ein gattungsgemäßes Flächenmaterial bereitzustellen, bei welchem elastischen Bereiche und nicht-elastische Bereiche verhältnismäßig einfach und zuverlässig dargestellt werden können.

Als Lösung wird einerseits ein Verfahren zur Herstellung eines Flächenmaterials mit elastischen und nicht-elastischen Bereichen sowie mit einem textilen Charakter zumindest auf einer Oberflächenseite vorgeschlagen, bei welchem ein Vliesmaterial und ein elastischer Film im wesentlichen flächig durch Aufeinanderpressen miteinander verbunden werden, wobei die Anpresskräfte in den elastischen Bereichen geringer als in den nicht-elastischen Bereichen sind und welches sich dadurch auszeichnet, dass das Vliesmaterial in einer Kuschier- oder Verbindungseinheit, welche unmittelbar hinter einer Extrusionseinheit für den elastischen Film angeordnet ist, mit dem noch plastischen Film durch leichtes Aneinanderdrücken verbunden werden, wobei durch ein verstärktes Aneinanderpressen in ausgewählten Bereichen das Vliesmaterial bzw. dessen einzelne Fasern stärker in den noch plastischen Film gepresst werden, als dieses in den übrigen Bereichen geschieht und/oder dass das Vliesmaterial vor dem Verbinden mit dem elastischen Film mittels einer Verbindungseinrichtung in einem im Wesentlichen losen Verbund der Verbindungseinrichtung zugeführt wird.

Ebenso schlägt die Erfindung ein Flächenmaterial mit elastischen und nicht-elastischen Bereichen sowie mit einem textilen Charakter zumindest auf einer Oberfläche vor, wobei eine Vliesmateriallage und eine elastische Filmlage im Wesentlichen flächig miteinander verbunden sind, wobei die Verbindung in den elastischen Bereichen geringer als in den nicht-elastischen Bereichen ist und wobei sich das Flächenmaterial dadurch auszeichnet, dass die Bindungsstärke zwischen den Vliesfasern und der elastischen Filmlage in den nicht-elastischen Bereichen zumindest 1,5-mal so groß ist wie die Bindunssstärke zwischen den Vliesfasern und der elastischen Filmlage in den elastischen Bereichen.

Hierbei geht die Erfindung von der Grunderkenntnis aus, dass durch eine flächige Verbindung, welche letztlich die Mehrzahl der Vliesfasern mit der elastischen Filmlage verbindet, eine gleichförmige und betriebssichere Befestigung der Vliesmateriallage an der elastischen Filmlage sowie eine Verfestigung der Vliesmateriallage an sich gewährleistet werden kann. Durch die Variation der Bindungsstärke bzw. Bindungsdichte können dann ohne weiteres elastische und nicht-elastische Bereiche ausgebildet weiden. In Abweichung von dei WO 2001/53076 A1 sind bei der erfindungsgemäßen flächigen Verbindung somit auf der Seite der Vliesmateriallage, welche der elastischen Filmlage zugewandt ist, die Mehrzahl der einzelnen Vliesfasern mit der elastischen Filmluge verbunden, während bei der WO 2001/53076 A1 eine derartige Verbindung, insbesondere in den elastischen Bereichen, lediglich an diskreten Schweißpunkten zu finden ist.

Bei einer derartigen Ausgestaltung kann insbesondere auf ein "Prestretching", also ein Aktivieren der elastischen Bereiche, wie dieses aus dem übrigen Stand der Technik bekannt ist, verzichtet werden, da die geringere Verbindung in den elastischen Bereichen eine unterschiedliche Versteifung der elastischen Filmlage bedingt, so dass die elastische Filmlage in den nicht-elastischen Bereichen durch die Vliesmateriallage derart stabilisiert ist, dass sich in gewünschter Weise elastische und nicht-elastische Bereiche ausbilden. Andererseits versteht es sich, dass unter gegeben Umständen ein Prestretching vorgenommen werden kann, wobei jedoch die hier für benötigten Kräfte um Größenordnungen geringer sind als dieses bei Anordnungen nach dem Stand der Technik der Fall ist, bei welchen die Vlieslage und die elastische Filmlage großflächig mit gleicher Kraft miteinander verbunden und die elastischen Bereiche durch ein Prestretching bereit gestellt werden.

Im vorliegenden Zusammenhang wird jeder Bereich des Flächenmaterials, welcher elastischer als die mittlere Gesamtelastizität des gesamten Flächenmaterials ist, als elastischer Bereich bezeichnet, während jeder Bereich, dessen Elastizität geringer als die mittlere Gesarntelastizität ist, als nicht elastischer Bereich bezeichnet wird. Vorzugsweise ist die jeweilige Ausdehnung der elastischen Bereichen bzw. der nicht-elastischen Bereiche weinigstens so groß wie die mittlere Faserlänge der einzelnen Fasern des Vliesmaterials, insbesondere kann die jeweilige Ausdehnung der elastischen Bereichen bzw. der nicht-elastischen Bereiche zumindest doppelt so groß wie die mittlere Faserlänge der einzelnen Fasern des Vliesmaterials sein. Kumulativ bzw. alternativ hierzu beträgt die jeweilige Ausdehnung der elastischen Bereichen bzw. der nicht-elastischen Bereiche vorzugsweise zumindest 10 mm. Es versteht sich, dass in den elastischen bzw. nicht-elastischen Bereichen die Elastizität ohne weiteres auch Schwankungen, insbesondere auch regelmäßigen Schwankungen, wie sie beispielsweise durch Elastifizierungsprozesse bedingt sein können, unterliegen können. Hierbei werden jedoch durchweg in den nicht-elastischen Bereichen Teilbereiche mit im Vergleich zu anderen Teilbereichen des entsprechenden nicht-elastischen Bereichs geringerer Elastizität eine geringere Elastizität aufweisen als Teilbereiche der elastischen Bereiche mit im Vergleich zu anderen Teilbereichen des entsprechenden elastischen Bereichs geringerer Elastizität. Ebenso werden durchweg in den elastischen Bereichen Teilbereiche mit im Vergleich zu anderen Teilbereichen des entsprechenden elastischen Bereichs höherer Elastizität eine höhere Elastizität aufweisen als Teilbereiche der nicht-elastischen Bereiche mit im Vergleich zu anderen Teilbereichen des entsprechenden nicht-elastischen Bereichs höheren Plastizität. Vorzugsweise weisen selbst in den elastischen Bereichen Teilbereiche mit im Vergleich zu anderen Teilbereichen des entsprechenden elastischen Bereichs geringerer Elastizität eine höhere Elastizität auf als Teilbereiche der nicht-elastischen Bereiche mit im Vergleich zu anderen Teilbereichen des entsprechenden nicht-elastischen Bereichs höheren Elastizität. Ebenso werden in der Regel durchweg selbst Teilbereiche der nicht-elastischen Bereiche, die eine höhere Elastizität als die sie umgebenden Teilbereiche des entsprechenden nicht-elastischen Bereichs aufweisen, eine geringere Elastizität aufweisen als die Teilbereiche eines anliegenden elastischen Bereichs, die eine niedrigere Elastizität aufweisen als die sie umgebenden Teilbereiche des elastischen Bereichs. Hierbei versteht es sich, dass lokale Spitzen, deren räumliche Ausdehnung verhältnismäßig klein ist und die mithin das makroskopische Verhalten des Flächenmaterials nur unwesentlich beeinflussen, keine Rolle spielen.

Vorzugsweise ist die elastische Lage durchgängig ausgebildet und kann somit entsprechend durchgängig die Vliesmateriallage stabilisieren. Dieses ermöglicht den Einsatz besonders weicher bzw. streckfähiger Vliesmateriallagen, wobei insbesonders letztere Eigenschaft in den elastischen Bereichen für gute Elastizitäten sorgt. Dementsprechend eignet sich vorliegende Erfindung insbesondere für Flächemnaterialien, bei denen in Querrichtung das Längenverhältnis von elastischen Bereichen zu nicht-elastischen Bereichen größer 1 : 2. insbesondere größer 1,2 : 2 bzw. größer 1,3 : 2 oder gar größer 1,4 : 2, ist. Bei Anordnungen mit geringerem Längenverhältnis zwischen elastischen und nicht-elastischen Bereichen werden nicht-elastischen Bereiche in der Regel nicht mit einer elastischen Lage versehen, die an sich sehr kostspielig und in diesen Bereichen eigentlich redundant ist. In derartigen Auwendungsfällen wird in der Regel eine mehrstückige Anordnung, bei welchen ein elastischer Streifen, beispielsweise mittels eines kleinen Überlapps, an zwei nicht-elastische Streifen angesetzt wird, so dass ebenfalls ein Flüchenmaterial mit elastischen und nicht-elastischen Bereichen vorliegt, dieses jedoch aus im Querschnitt völlig unterschiedlich aufgebauten, aneinander gesetzten Streifen besteht.

Hierbei eignet sich die vorliegende Erfindung dementsprechend insbesondere für Anordnungen, bei denen das Flächenmaterial einstückig zur Anwendung kommt. Dieses kann beispielsweise bei Windelohren oder Windelverschlussbändem der Fall sein, die über verhältnismäßig schmale nicht-elastische Bereiche verfügen, in welchen Befestigungseinrichtungen zur Befestigung an einer Windel vorgesehen sind und zwischen denen ein verhältnismäßig großer elastischer Bereich vorgesehen sein soll, der dem Tragekomfort, einer Größenanpassung bzw. einem einfachen An- oder Ablegen der Windel dient. Als Befestigungseinrichtungen können klebend wirksame Befestigungsmöglichkeiten oder auch mechanische Befestigungsmöglichkeiten sowie alle übrigen bekannten Befestigungsmöglichkeiten zur Anwendung kommen. Sind dies wieder lösbar. wie beispielsweise Haken-Schlaufen-Verbindungen oder taktil wirksame Klebstoffe, so können diese insbesondere im Befestigungsbereich eines entsprechend wirksamen Windelohrs oder Windelverschlussbands zu Anwendung kommen. Halten diese Permanent, wie beispielsweise Heißkleber oder Verschweißungen, so eignen sich diese insbesondere für den Permanentbereich eines entsprechend wirksamen Windelohrs oder Windelverschlussbands, welcher zum permanenten Verbleib an der entsprechenden Windel gedacht ist.

Eine Variation der Anpresskräfte lässt sich besonders einfach realisieren, wenn die Anordnung aus Vliesmaterial und elastischem Film, vorzugweise unmittelbar hinter einer Kaschiereinheit, über eine Kalanderwalze mit einer Oberflächenstruktur aufeinander gepresst werden, wobei vorzugsweise radial hervorstehende Bereiche der Kalanderwalze die nicht-elastischen Bereiche bilden. Dieses geschieht dadurch, dass die hervorstehenden Bereiche der Kalanderwalze das Vliesmaterial verhältnismäßig stark mit dem elastischen Film verpressen. Hierdurch erhöht sich die Bindung zwischen der Vliesmateriallage und der elastischen Filmlage, so dass diese erheblich miteinander versteift werden. Dieses führt dann zur Ausbildung der nicht-elastischen Bereiche.

In einer bevorzugten Verfahrungsführung werden das Vliesmaterial und der elastische Film thermisch miteinander verbunden. Dieses kann vorzugsweise durch Extrusionslaminieren oder Verschweißen erfolgen, wobei ersteres bevorzugt wird.

Insbesondere kann dass Vliesmaterial in einer Kaschier- oder Verbindungseinheit, welche unmittelbar hinter einer Extrusionseinheit für den elastischen Film angeordnet ist, mit dem noch plastischen Film durch leichtes Aneinanderdrücken verbunden werden, wobei durch ein verstärktes Aneinanderpressen in ausgewählten Bereichen das Vliesmaterial bzw. dessen einzelne Fasern stärker in den noch plastischen Film gepresst werden, als dieses in den übrigen Bereichen geschieht. Hierbei kann das Anpressen auch in einem separaten Schritt erfolgen, während der elastische Film in einem plastischen Zustand ist. Auf diese Weise kann das so gebildete Flächenmaterial in bestimmten Bereichen ohne weiteres derart versteift werden, dass nicht-elastische Bereiche gebildet werden.

Alternativ ist es möglich, das Vliesmaterial und den elastischen Film miteinander zu verkleben, wobei durch die unterschiedliche Anpresskräften entsprechend unterschiedlich große Bindungen erzielt werden können.

Vorzugsweise wird als Vliesmaterial NonWoven-Material gewählt. Ein derartiges Material kann einem Flächenmaterial ohne weiteres zumindest auf der Oberflächenseite, uuf der es angeordnet ist, einen textilen Charakter verleihen. Darüber hinaus können die den Eigenschaften eines derartigen Materials in großen Grenzen frei gewühlt werden, so dass insbesondere eine gute Verarbeitbarkeit vor dem Verbinden des Vliesmaterials mit dem elastischen Film gewährleistet werden kann, ohne dass das NonWoven derart eigenstabil ist, dass es eine Ausbildung nicht-elastischer Bereiche erschwert.

Insbesondere kann das Vliesmaterial vor dem Verbinden mit dem elastischen Film in einem im Wesentlichen losen Verbund der Verbindungseinrichtung zugeführt werden. Insofern ist es kumulativ bzw. alternativ hierzu von Vorteil, wenn die Bindungsstärke der Vliesfasern untereinander zumindest eine Größenordnung unter der Bindungsstärke zwischen den Vliesfasern und der elastischen Filmlage liegt. Insofern ist es von Vorteil, wenn erst der elastische Film das Vliesmaterial stabilisiert bzw. im gewünschten Maße verfestigt. Bei einer derartigen Ausgestaltung kommt es zur Ausbildung einer vorteilhaften Wechselwirkung zwischen den an sich nicht dehnbaren, aber verhältnismäßig kurzen Einzelfasern des Vlieses, welche dem elastischen Film seine Elastizität nehmen, wenn sie verhältnismäßig stark, insbesondere wenn sie über eine verhältnismäßig große Länge bzw. an mehrere Punkten, mit dem elastischen Film verbunden sind. Andererseits führt erst der elastische Film dazu, dass das Vliesmaterial in sich stabilisiert wird, um insbesondere unter Bedingungen beim Endverbraucher ausreichend eigenstabil und in sich verfestigt zu erscheinen. Der lose Verbund des Vliesmaterial ermöglicht darüber hinaus, insbesondere in den elastischen Bereichen, dass sich der Film elastisch dehnen kann.

Vorzugsweise ist die Bindungsstärke zwischen den Vliesfasern und der elastischen Filmlage in den nicht-elastischen Bereichen zumindest 1,5-mal so groß wie die Bindungsstärke zwischen den Vliesfasern und der elastischen Filmlage in den elastischen Bereichen. Bereits ein derartiger, verhältnismäßiger geringer Unterschied in der Bindungsstärke führt dazu, dass die elastischen Bereiche sich bevorzugt dehnen, wenn das erfindungsgemäße Flächenmaterial einer Belastung unterzogen wird.

Je nach konkreter Ausführungsform kann der elastische Film bzw. die elastische Filmlage Löcher oder Öffnungen aufweisen, welche vorzugsweise kleiner als die mittlere Faserlänge der Fasern des Vliesmaterial sind. Derartige Löcher oder Öffnungen können einerseits einer Belüftung dienen. Sie führen darüber hinaus zu einer Materialersparnis und können dazu dienen, dass Elastizitätsmodul in Maschinenrichtung bzw. in Querrichtung zu beeinflussen. Es versteht sich, dass derartige Öffnungen auch unabhängig von den übrigen Merkmalen vorliegender Erfindung für ein Flächenmaterial mit einer Vliesmateriallage und einer elastischen Filmlage entsprechend vorteilhaft sein können.

Im vorliegenden Zusammenhang bezeichnet der Begriff "Maschinenrichtung" eine Ausrichtung in der Ebene des Flächenmaterials, welche parallel zur Durchlaufrichtung des Flächenmaterials durch die Verbindungseinnchtung gerichtet ist. Der Bergriff "Querrichtung" bezeichnet dementsprechend eine senkrecht hierzu gebildete Richtung, die ebenfalls in der Ebene des Flächenmaterials liegt.

Insbesondere in den nicht-elastischen Bereichen, in denen das Flächenmaterial mit wesentlich steiferen Komponenten, wie beispielsweise Hakenmaterialien zur Bildung eines Verschlusselementes oder auch dem Backsheet einer Windel, verbunden werden soll, kann es von Vorteil sein, das Flächenmaterial über die Steifigkeit, welche durch die unterschiedlichen Anpresskräfte bedingt ist, hinaus noch weiter zu versteifen. Durch eine derartige Zusatzversteifung können insbesondere Peel-Off-Effekte bzw. ein Abscheren des erfindungsgemäßen Flächenmaterials von den steiferen Komponenten minimiert werden.

Dieses kann beispielsweise durch eine zusätzliche, versteifende Lage geschehen. Vorzugsweise ist diese Lage nicht durchgehend ausgebildet, so dass durch sie das übrige Verhalten des Flächenmaterials nur unwesentlich beeinflusst wird. Auch kann die Lage in das Flächenmaterial eingearbeitet werden, was insbesondere ohne weiteres während der Herstellung geschehen kann. Hierbei ist eine nicht durchgängig Zusatzlage von besonderem Vorteil, du dann die Verbindung der Flächenmateriallagen untereinander, insbesondere mithin die Verbindung zwischen elastischer Lage und Vliesmateriallage nicht beeinträchtigt wird. Ebenso kann diese Zusatzlage auch zur Gänze in eine andere Lage, insbesondere in die elastische Filmlage eingearbeitet werden.

Vorzugsweise ist die Zusatzlage als ein Netz oder Gitter ausgebildet. Besonders geeignet erscheint ein Netz au Polyethylen (PE) und/oder Polypropylen (PP), welches insbesondere im Vergleich zu der elastischen Filmlage aber auch im Vergleich zu dem Flächenmaterial auch in seinen nicht-elastischen Bereichen wesentlich steifer bzw. unelastischer ausgebildet werden kann. Außerdem können die vorgenannten Materialien einfach und betriebssicher mit den übrigen Materialien des erfindungsgemäßen Flächenmaterials verbunden werden.

Alternativ bzw. kumulativ kann eine derartige Versteifung durch eine Klebstoffbeschichtung oder auch durch eine in das Vlies eingedrungene Klebstoffschicht realisiert werden. Ebenso ist eine Versteifung durch eine thermische Verfestigung, beispielsweise durch leichtes Anschmelzen der Vliesfasern, umzusetzen. Auch durch Ultraschallbonds bzw. Ultraschallschweißpunkte kann einfach und betriebssicher entsprechend eine Versteifung bereitgestellt werden. Hierbei kann die versteifende Behandlung vor oder nach dem Zusammenfügen der Lagen des Flächenmaterials vorgenommen werden.

Je nach konkreter Ausgestaltung des erfindungsgemäßen Flächenmaterials bzw. des erfindungsgemäßen Herstellungsverfahrens können die stärker verpressten bzw. die nicht-elastischen Bereiche eine andere Dicke aufweisen als die übrigen Bereiche des Flächenmaterials. Hierbei ist zu berücksichtigen, dass es in diesem Zusammenhang insbesondere auf den subjektiven Eindruck Dritter, als den subjektiven Eindruck beispielsweise von Kunden, ankommt, welcher festlegt, ob diesbezüglich Maßnahmen getroffen werden müssen.

Sollte dieses der Fall sein, so wird einerseits vorgeschlagen, auf das Flächenmaterial eine weitere Vliesmateriallage aufzukaschieren. Hierdurch können Dickenunterschiede derart kaschieren, dass sie das subjektive Empfinden Dritter nicht mehr erheblich stören. Je nach Erfordernissen kann die weitere Vliesmateriallage lediglich in Teilbereichen, insbesondere in den nicht-elastischen Bereichen, mit dem übrigen Flächenmaterial verbunden sein. Als Verbindung stehen sämtliche Verbindungsarten, wie Verklebungen, Verschweißungen, beispielsweise mit Ultraschall, oder sonstige Verbindungen, zur Verfügung. Auch kann die weitere Vliesmateriallage eine variierende Dicke aufweisen und beispielsweise in den Bereichen, in denen das übrige Flächenmaterial dünner ist, dicker ausgebildet sein.

In diesem Zusammenhang sei erwähnt, dass auch die übrigen Lagen des erfindungsgemäßen Flächenmaterials, insbesondere vor dem Zusammenfügen, variierende Dicken aufweisen können. So kann beispielsweise eine Vliesmateriallage in den nicht-elastischen Bereichen dicker gewählt sein als in den elastischen Bereichen. Ebenso ist es denkbar, eine Vliesmateriallage vor dem Zusammenfügen in den elastischen Bereichen zu verdünnen, was beispielsweise auch durch einen vorgeschalteten Kompressionsschritt realisiert werden kann. Es versteht sich, dass derartige Maßnahmen auch für die elastische Filmlage vorgesehen sein können.

Andererseits kann in dem dünneren Bereich durch eine eingebrachte Struktur, was beispielsweise durch eine nachgeschaltete bzw. zum Verpressen genutzte Strukturwalze realisiert werden kann, ein Höhenausgleich zu den weniger verpressten und mithin dickeren Bereichen geschaffen werden. Hierdurch kann beispielsweise das noch formbare elastische Material mit einem dreidimensionalen Muster versehen werden, welches einem Dritten einen dickeren Eindruck vermittelt. Wird die Struktur geeignet fein strukturiert gewählt, so erfolgt in diesen Bereichen insgesamt immer noch eine ausreichende Verdichtung, welche die erfindungsgemäßen Vorteile bedingt.

Ebenso kann der Dickenausgleich durch eingebrachtes Material, insbesondere beispielsweise durch die vorgenannte Zusatzlage realisiert werden.

Weitere Vorteile, Ziele und Eigenschaften vorliegender Erfindung werden anhand der nachfolgenden Beschreibung anliegender Zeichnung erläutert. In der Zeichnung zeigen:
- Figur 1: eine erfindungsgemäße Verbindungseinrichtung;
- Figur 2: eine schematische Darstellung eines erfindungsgemäßen Flächenmaterials im Schnitt;
- Figur 3: eine schematische Darstellung eines weiteren erfindungsgemäßen Flächenmaterials im Schnitt;
- Figur 4: eine schematische Darstellung eines weiteren erfindungsgemäßen Flächenmaterials im Schnitt;
- Figur 5: eine schematische Darstellung eines weiteren erfindungsgemäßen Flächenmaterials im Schnitt;
- Figur 6: eine schematische Darstellung eines weiteren erfindungsgemäßen Flächenmaterials im Schnitt;
- Figur 7: eine schematische Darstellung eines weiteren erfindungsgemäßen Flächenmaterials im Schnitt;
- Figur 8: eine schematische Darstellung eines weiteren erfindungsgemäßen Flächenmaterials im Schnitt;
- Figur 9: eine schematische Darstellung eines weiteren erfindungsgemäßen Flächenmaterials im Schnitt;
- Figur 10: eine schematische Darstellung eines weiteren erfindungsgemäßen Flächenmaterials im Schnitt;
- Figur 11: eine Aufsicht auf eine elastische Filmlage mit exemplarische Öffnungen; und
- Figur 12: eine weitere Aufsicht auf eine elastische Filmlage mit exemplarische Öffnungen.

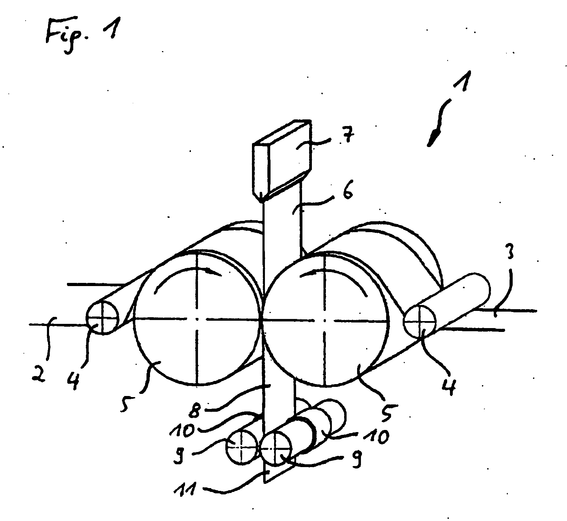

Die in Figur 1 dargestellte Verbindungseinrichtung 1 kaschiert Vliesmaterial 2, 3 über Vorlegewalzen 4 und Kaschierwalzen 5, welche ggf. temperiert sein können, auf einen elastischen Film 6, welcher unmittelbar aus einer Extrudiereinrichtung 7 bereit gestellt wird, zu einem elastischem Flächenmaterial 8. Die Verbindung zwischen dem Vliesmaterial 2, 3 und dem elastischen Film 6 erfolgt hierbei thermisch, wobei das Vliesmaterial 2, 3 mit dem elastischen Film 6 in Kontakt kommt, wenn dieser sich noch in seinem plastischen Zustand befindet. Hierbei versteht es sich, dass in einer alternativen Ausführungsform auch eine andere Verbindung, beispielsweise ein Klebeverbindung, vorgesehen sein kann.

Den Walzen 5 nachgeschaltet, sind zwei Kalanderwalzen 9 vorgesehen, die jeweils radial hervorstehenden Bereiche 10 aufweisen, welche ausgewählte Bereiche des elastischen Films 8 mit Anpresskräften beaufschlagen, so dass das Vliesmaterial 2, 3 dort stärker mit dem elastischem Film 6 verbunden wird. Auf diese Weise wird ein Flächenmaterial 11 bereitgestellt, welches elastische Bereiche und 12 und nicht-elastische Bereiche 13 aufweist. Auch bei anderen Verbindungen, wie beispielsweise bei Klebeverbindungen, können auf diese Weise entsprechend alternierende Bereiche geschaffen werden. Im Übrigen versteht es sich, dass alternativ bzw. kumulativ auch die Walzen 5 eine entsprechende Profilierung aufweisen können. Auch können die Kalanderwalzen 9 derart justiert sein, dass sie auf sämtliche Bereiche Anpresskräfte ausüben, letztere jedoch in den Bereichen 10 stärker als in den übrigen Bereichen sind.

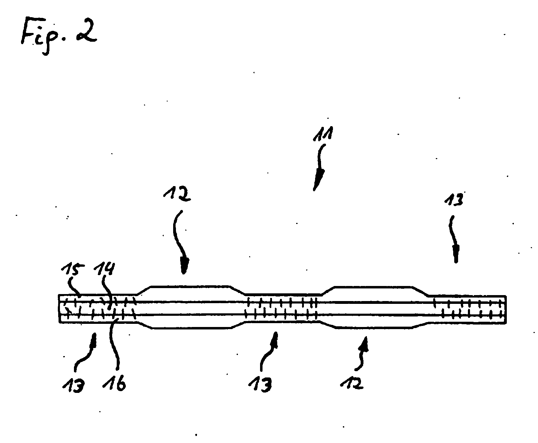

Das Flächenmaterial 11 umfasst, wie insbesondere anhand der Figur 2 ersichtlich, eine zentrale elastische Filmlage 14, welche durch den elastischen Film 6 dargestellt wird, und auf beiden Oberflächenseiten jeweils eine Vliesmateriallage 15, 16, welche jeweils durch das Vliesmaterial 2, 3 dargestellt werden. Durch die radial hervontehenden Bereiche 10 der Kalanderwalzen 9 sind die Vliesmateriallagen 15, 16 derart fest mit der elastischen Filmlage 14 verbunden, dass deren Elastizität in den nicht-elastischen Bereichen 13 nicht zur Geltung kommen kann, was in Figur 2 durch kurze Strichlinien verdeutlicht ist.

Je nach konkreter Ausgestaltung kann auch lediglich eine Kalanderwalze vorgesehen sein. Ebenso ist es denkbar, dass die Kalanderwalze mit einer nicht profilierten Walze zusammenwirkt. In einer bevorzugten Ausführungsform sind die elastischen Bereiche 12 und die nicht-elastischen Bereiche 13 streifenartig in Maschinenrichtung, also mit geradlinigen Begrenzungslinien ausgerichtet. Andererseits können auch wellenförmige Begrenzungslinien oder einzelne diskrete Muster oder ähnliches als elastische Bereiche bzw. nicht-elastische Bereiche ausgebildet werden. Es versteht sich unmittelbar, dass statt des vor stehend beshriebenen Herstellungsverfahrens die elastische Filmlage und/oder die Vliesmateriallagen auch im Nachhinein erwärmt werden können, um nicht-elastische Bereiche zu schaffen. Ebenso können die Lagen miteinander verklebt werden, wobei durch unterschiedliche Anpresskräfte unterschiedliche Bindungsstärken gewährleistet werden können.

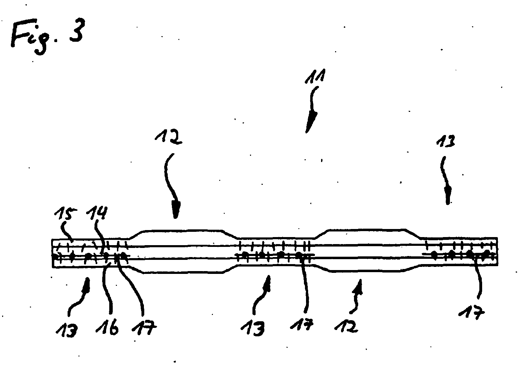

Die in den Figuren 3 bis 10 dargestellten Ausführungsbeispiele entsprechen im Wesentlichen dem Ausführungsbeispiel nach Figur 2, so dass identisch wirksame Baugruppen auch identisch bezeichnet sind. Auch diese Ausführungsbeispiele können, mit geringfügigen Modifikationen, ohne weiteres auf der in Figur 1 dargestellten Verbindungseinrichtung hergestellt werden.

Bei dem in Figur 3 dargestellten Ausführungsbeispiel ist zur Versteifung in das Flächenmaterial 11 ein PE-Netz 17 eingearbeitet worden, indem dieses bei der Herstellung in dem entsprechenden Bereich jeweils auf das Vliesmaterial 3 (siehe Figur 1) aufgelegt wurde, bevor dieses mit dem elastischen Film 6 in Kontakt gebracht wurde. Es versteht sich, dass das PE-Netz 17 ggf. auch beidseits vorgesehen sein kann. Ebenso muss das PE-Netz 17 nicht in allen bzw. nicht in einem ganzen nicht-elastischen Bereich 13 vorgesehen sein. Darüber hinaus ersteht es sich, dass das PE-Netz 17 auch zur Gänze in den elastischen Film 6 eingearbeitet werden kann, was beispielsweise durch eine doppelte Extrudiereinrichtung 7 oder dadurch, dass das PE-Netz 17 letztere durchläuft, realisiert werden kann. Ebenso kann das Netz statt aus PE auch aus anderen Materialien, beispielsweise aus PP, oder aus Materialkombinationen gebildet werden.

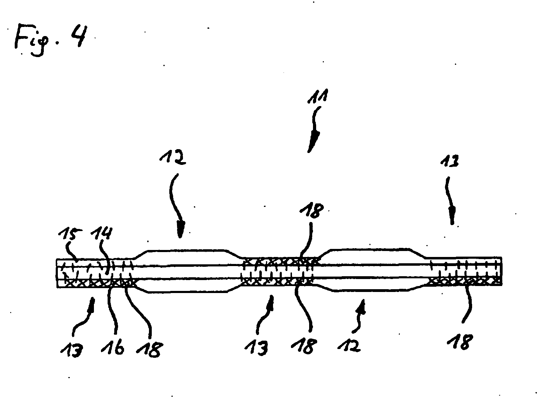

Statt des PE-Netzes 17 kann in den nicht-elastischen Bereichen eine Versteifung durch eine Klebstofffixierung 18 vorgesehen sein, wie dieses anhand des Ausführungsbeispiels in Figur 4 exemplarisch erläutert ist. Wie dort dargestellt, ist nicht jede Vliesmateriallage 15, 16 in den nicht-elastischen Bereichen 13 entsprechend durch die Klebstofffixierung stabilisiert. Die Stabilisierung erfolgt jeweils dort, wo sie entsprechend des Einsatzzweckes des Flächenmaterials 11 erforderlich erscheint. Bei diesem Ausführungsbeispiel ist die Klebstofffixierung durch einen Klebstoff realisiert, der nachträglich in flüssiger Form aufgebracht und dann in die jeweilige Vliesmateriallage 15, 16 eingedrungen und sich dort verfestigt hat. In alternativen Ausführullgsformen kann auch Klebstoff zuvor aufgebracht oder als eigenständige Klebstoffschicht vorgesehen werden.

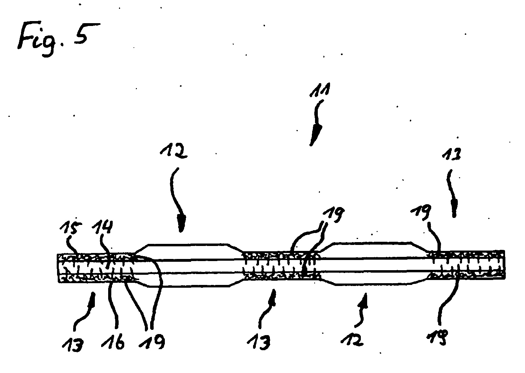

Bei dem in Figur 5 dargestellten Ausführungsbeispiel erfolg die Versteifung durch eine thermische Verfestigung der Vliesmateriallagen 15, 16 in thermisch verfestigten Bereichen, indem die Vliesmateriallagen 15, 16 in diesen Bereichen vor, während und/oder nach dem Zusammenfugen der Lagen entsprechend erwärmt werden, so dass die Bindungskräfte zwischen den Fasern ansteigen. Es versteht sich, dass auch bei diesem Ausführungsbeispiel nicht sämtliche nicht-elastischen Bereiche 13 entsprechend versteift werden müssen.

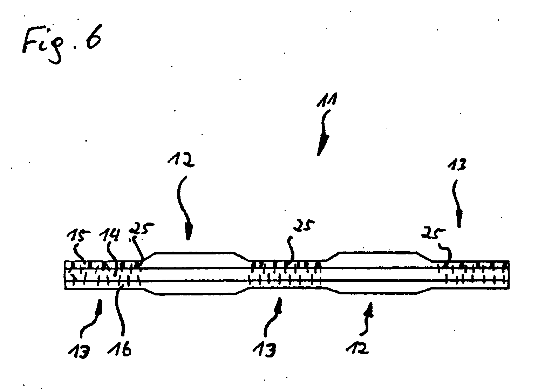

Eine Versteifung ist bei dem in Figur 6 dargestellten Ausführungsbreispiel durch Ultraschallpunkte 25 realisiert, die entsprechend vor, während oder nach dem Zusammenfügen in die Vliesmateriallage 15 eingebracht wurden. Auch hier versteht es sich, dass die zusätzliche Versteifung jeweils dort vorgesehen wird, wo sie sinnvoll bzw. notwendig ist.

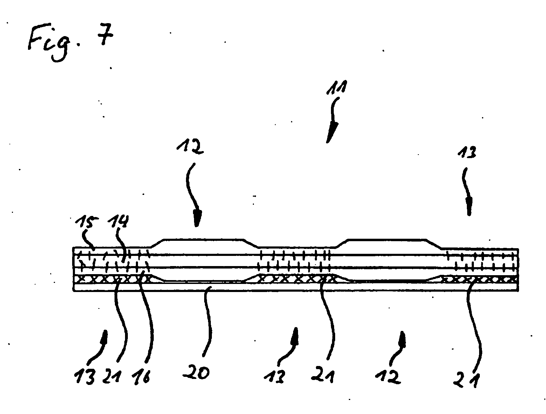

Durch die unterschiedlichen Anpresskräfte kann es zu unterschiedlich dick ausgebildeten Bereichen des Flächemnaterials 11 kommen, was möglicherweise von Kunden als störend empfunden wird. Um diesem entgegen zu wirken, ist bei dem Ausführungsbeispiel nach Figur 7 eine weitere Vliesmateriallage 20 über Klebstoffschichten 21 auf das Flächemnaterial 11 aufkaschiert. Hierbei dienen insbesondere die Klebstoffschichten 21 als Dickenausgleich, wobei sie jedoch auch eine zusätzliche Versteifung in den nicht-elastischen Bereichen bedingen. In einer alternativen Ausführungsform kann jedoch auch auf den Dickenausgleich durch die Klebstoffschichten 21 verzichten werden, wenn schon die weitere Vliesmateriallage 20 die Dickenunterschiede ausreichend kaschiert.

Dementsprechend ist es auch denkbar, dass der Klebstoff entsprechend des Ausführungsbeispiels nach Figur 4 ganz oder teilweise in die Vliesmateriallagen 15, 16 eindringt und auf diese Weise einerseits verfestigend und andererseits die weitere Vliesmateriallage 20 befestigend wirkt.

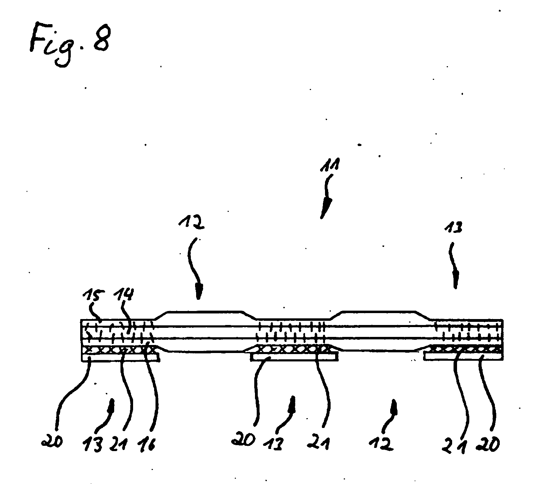

Wie das Ausführungsbeispiel nach Figur 8 andeutet, muss die Vliesmateriallage 20 nicht zwingend durchgehend ausgebildet sein. Sie kann insbesondere in den elastischen Bereichen 12 auch weggelassen werden, so dass die hervorragende haptische Qualität der Vlieslagen 15, 16, die unmittelbar mit der zentralen elastischen Filmlage verbunden sind, zur Geltung kommt. Hierdurch wird einerseits Material eingespart. Andererseits erfolgt der oben angesprochene Dickenausgleich hierdurch ohne weiteres unmittelbar. In diesem Zusammenhang versteht es sich, dass die streifenförmig aufgebrachte, weitere Vliesmateriallage 20 insbesondere auch gemeinsam mit den beiden Vliesmateriallagen durch den Spalt der Kalanderwalzen 9 geführt werden kann und auf diese Weise bereits unterschiedliche Anpressdrücke bedingt. Insofern kann bei einer geeigneten Verfahrensfuhrung auf eine Strukturlerung der Kalanderwalzen 9 oder ganz auf die Kalanderwalzen 9 verzichtet werden.

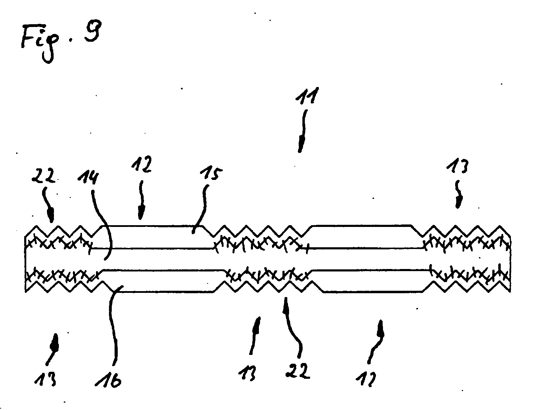

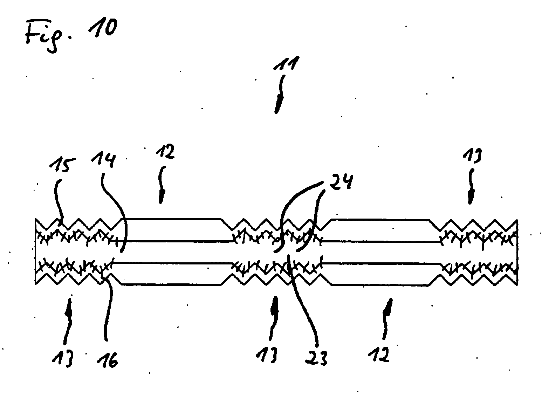

Die Dickenunterschiede können, wie Figuren 9 und 10 darstellen, auch durch Rippen 22 bzw. durch Bereiche 23, in denen Material des Films 6 angehäuft ist, und Bereiche 24, in denen weniger Material des Films 6 vorgesehen ist, kaschiert werden. Derartige Profilierungen können beispielsweise über die Walzen 5 bzw. über die Kalanderwalzen 9 (siehe Figur 1) bereitgestellt werden. Hierbei ist die Dichte der Profilierung derart gewählt, dass in den nicht-elastischen Bereichen 13 insgesamt eine ausreichende Verdichtung vorliegt und entsprechend erhöhte Anpresskräfte garantiert bleiben. Diese kann auch bei Rippen oder Materialanhäufungen insbesondere dadurch gewährleistet werden, dass bei dem Verpressen der entsprechenden Profilierung ausreichend Querkräfte in das Material eingetragen werden.

Es versteht sich, dass die Bereiche 23 und 24 nicht zwingend linear, insbesondere in Maschinenrichtung linear, ausgebildet sein müssen, wie dieses in Figuren 9 und 10 dargestellt ist. Hier kann jede geeignete Profilierung, insbesondere beispielsweise in Form von Punkten, die besonders verdichtet sind, oder aber auch in Form von Punkten, die gerade nicht verdichtet sind, gewählt werden. Hierbei wird die Profilierung insbesondere derart gewählt, dass die Gesamtverdichtung in den nicht-elastischen Bereichen 13 ausreichend hoch ist, um die erfindungsgemäßen Vorteile umsetzen zu können.

Hierbei versteht es sich, dass auch in den elastischen Bereichen 12 eine Profilierung vorgesehen sein kann, wobei jedoch die Bindung in den elastischen Bereichen zwischen Vliesmateriallagen und elastischer Filmlage ausreichend geringer ist, um die gewünschten Elastizitätsunterschiede zu gewährleisten. Dieses kann beispielsweise durch entsprechend geringere Anpressdrücke oder aber durch einen Verzicht auf zusätzliche Maßnahmen, wie beispielsweise dem Einbringen von Klebstoff gewährleistet werden. Ebenso kann dieses dadurch gewährleistet werden, dass die Profilierung in den elastischen Bereichen späten, beispielsweise nach einem vollständigen Erkalten der elastischen Filmlage 14, eingebracht wird. Auf diese Weise ist ihr Einfluss auf die Bindungsstärke nur äußerst gering und die Profilierung hat eher Form gebenden und Oberfläche gestaltenden Charakter.

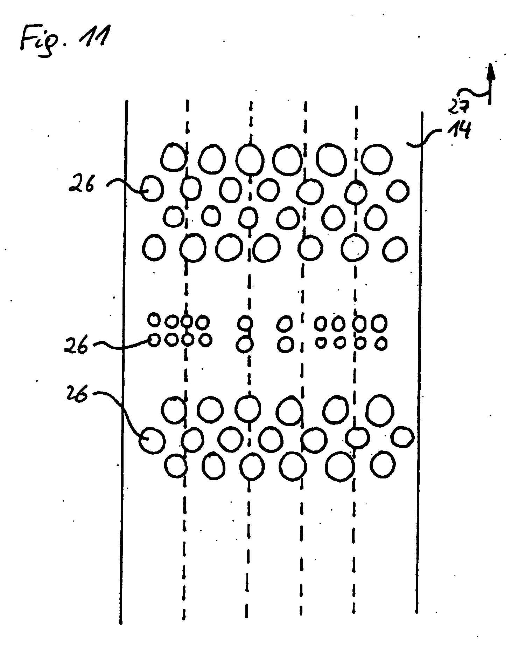

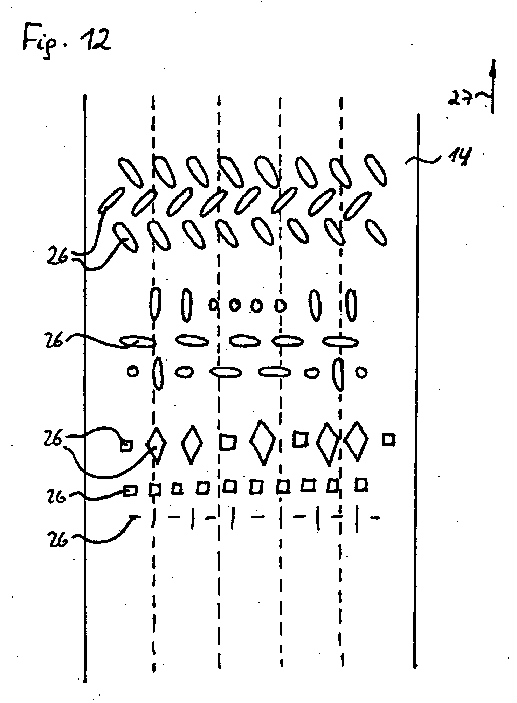

Die in den beiden Aufsichten nach Figuren 11 und 12 dargestellten Filmlagen sind exemplarisch mit Öffnungen 26 (lediglich exemplarisch beziffert) versehen, wobei die Maschinenrichtung, welch in Darstellungen nach Figuren 2 bis 10 senkrecht zur Zeichenebene liegt, durch den Pfeil 27 angedeutet ist. Wie unmittelbar ersichtlich können von dem Flächenmaterial beispielsweise quer zur Maschinenrichtung 27 als in Querrichtung Streifen abgeschnitten und beispielsweise als Windelverschlussband genutzt werden. Auch komplexere Trennformen, wie beispielsweise geschwungene Schnittlinien, sind ohne weiteres auch bei hohen Maschinengeschwindigkeiten zu realisieren.

Wie unmittelbar ersichtlich können insbesondere in Maschinenrichtung wiederkehrende Öffnungsanordnungen und auch Öffnungsausformungen zur Anwendung kommen. Als Öffnungen 26 im vorliegenden Sinnen können auch Schlitze oder Schnitte genutzt werden. Die Öffnungen 26 dienen hierbei insbesondere einer Erhöhung der Luftdurchlässigkeit. Bei geeigneter Ausformung und Anordnung kann durch sie auch das Dehnverhalten in sehr weiten Grenzen modifiziert werden.

Vorzugsweise sind die Öffnungen verhältnismäßig klein ausgebildet, so dass sie insbesondere unter der Vliesmasteriallage, besonders bei entspanntem Zustand der elastischen Filmlage, nicht oder kaum auffallen. Die Löcher können des Weiteren auf unterschiedlichste Weise in das Flächenmaterial 11 eingebracht werden.

Einerseits kann das Einbringen nach dem Kalandrieren bzw. nach dem Zusammenfügen der Lagen erfolgen. Hierdurch werden sicherlich häufig auch die Vlieslagen beeinflusst werden, wobei es denkbar ist, beispielsweise auf die elastische Filmlage abgestimmt Erwärmungsprozesse oder durch entsprechende chemische Prozesse lediglich die elastische Filmlage mit Öffnungen 26 zu versehen. Auch ist es möglich die Öffnungen 6 bereits bei der Extrusion bzw. vor oder mit dem Kaschieren der Lagen in den elastischen Film einzubringen.

### Bezugszeichenliste:

- 1: Verbindungseinrichtung
- 2: Vliesmaterial
- 3: Vliesmaterial
- 4: Vorlegewalzen
- 5: Kaschierwalzen
- 6: elastischer Film
- 7: Extrudiereinrichtung
- 8: elastisches Flächenmaterial
- 9: Kalanderwalzen
- 10: radial hervorstehende Bereiche
- 11: Flächenmaterial
- 12: elastische Bereiche
- 13: nicht-elastische Bereiche
- 14: zentrale elastische Filmlage
- 15: Vliesmateriallage
- 16: Vliesmateriallage
- 17: PE-Netz
- 18: Klebstofffixierung
- 19: thermisch verfestigter Bereich
- 20: weitere Vliesmateriallage
- 21: Klebstoffschicht
- 22: Rippen
- 23: Bereich mit mehr Material
- 24: Bereich mit wenigen Material
- 25: Ultraschallpunkt
- 26: Öffnung

## Patentansprüche

1. Verfahren zur Herstellung eines Flächenmaterials (11) mit elastischen und nicht-elastischen Bereichen (12, 13) sowie mit einem textilen Charakter zumindest auf einer Oberflächenseite, wobei ein Vliesmaterial (2, 3) und ein elastischer Film (6) im Wesentlichen flächig durch Aufeinanderpressen miteinander verbunden werden und wobei die Anpresskräfte in den elastischen Bereichen (12) geringer als in den nicht-elastischen Bereichen (13) sind, **dadurch gekennzeichnet, dass** das Vliesmaterial (2, 3) in einer Kaschier- oder Verbindungseinheit, welche unmittelbar hinter einer Extrusionseinheit für den elastischen Film (6) angeordnet ist, mit dem noch plastischen Film (6) durch leichtes Aneinanderdrücken verbunden wird wobei durch ein verstärktes Aneinanderpressen in ausgewählten Bereichen das Vliesmaterial (2, 3) bzw. dessen einzelne Fasern stärker in den noch plastischen Film (6) gepresst werden, als dieses in den übrigen Bereichen geschieht und/oder dass das Vliesmaterial (2, 3) vor dem Verbinden mit dem elastischen Film (6) mittels einer Verbindungseinrichtung (1) in einem im Wesentlichen losen Verbund der Verbindungseinrichtung (1) zugeführt wird.

2. Verfahren nach Anspruch 1, **dadurch gekennzeichnet, dass** die Anordnung (8) aus Vliesmaterial (2, 3) und elastischem Film (6) über eine Kalanderwalze (9) mit einer Oberflächenstruktur aufeinander gepresst werden, wobei vorzugsweise radial hervorstehende Bereiche (10) der Kalanderwalze (9) die nicht-elastischen Bereiche (13) bilden.

3. Verfahren nach Anspruch 1 oder 2, **dadurch gekennzeichnet, dass** das Vliesmaterial (2, 3) und der elastische Film (6) thermisch durch Extrusionslaminieren oder Verschweißen miteinander verbunden werden.

4. Verfahren nach einem der Ansprüche 1 bis 3, **dadurch gekennzeichnet, dass** das Vliesmaterial und der elastische Film miteinander verklebt werden.

5. Verfahren nach einem der Ansprüche 1 bis 4, **dadurch gekennzeichnet, dass** das Vliesmaterial ein NonWoven-Material ist.

6. Verfahren nach einem der Ansprüche 1 bis 5, **dadurch gekennzeichnet, dass** das Vliesmaterial (2, 3) beim Verbinden mit dem elastischen Film (6) durch den elastischen Film (6) stabilisiert wird.

7. Verfahren nach einem der Ansprüche 1 bis 6, **dadurch gekennzeichnet, dass** der elastische Film Löcher oder Öffnungen aufweist.

8. Verfahren nach Anspruch 7, **dadurch gekennzeichnet, dass** die Löcher oder Öffnungen kleiner als die mittlere Faserlänge der Fasern des Vliesmaterials (2, 3) sind.

9. Flächenmaterial (11) mit elastischen und nicht-elastischen Bereichen (12, 13) sowie mit einem textilen Charakter zumindest auf einer Oberflächenseite, mit einer Vliesmateriallage (15, 16) und einer elastische Filmlage (14), die im Wesentlichen flächig miteinander verbunden sind, wobei die Verbindung in dem elastischen Bereichen (12) geringer als in den nicht elastischen Bereichen (13) ist, **dadurch gekennzeichnet, dass** die Bindungsstärke zwischen den Vliesfasern und der elastischen Filmlage (14) in den nicht-elastischen Bereichen (13) zumindest 1,5-mal so groß ist wie die Bindungsstärke zwischen den Vliesfasern und der elastischen Filmlage (14) in den elastischen Bereichen (12).

10. FlÄchenmaterial nach Anspruch 9, **dadurch gekennzeichnet, dass** die Vliesmateriallage (15, 16) und die elastische Filmlage (14) thermisch miteinander verbunden sind.

11. Flächenmaterial nach Anspruch 9 oder 10 **dadurch gekennzeichnet, dass** die Vliesmateriallage und die elastische Filmlage miteinander verklebt sind.

12. Flächenmaterial nach einem der Ansprüche 9 bis 11, **dadurch gekennzeichnet**, das die Vliesmateriallage (15, 16) ein NonWoven-Material ist.

13. Flächenmaterial nach einem der Ansprüche 9 bis 12, **dadurch gekennzeichnet, dass** die Bindungsstärke der Vliesfasern untereinander zumindest eine Größenordnung unter der Bindungsstärke zwischen den Vliesfasern und der elastischen Filmlage (14) liegt.

14. Flächenmaterial nach einem der Ansprüche 9 bis 13, **dadurch gekennzeichnet, dass** die elastische Filmlage Löcher oder Öffnungen aufweist.

15. Flächenmaterial nach Anspruch 14, **dadurch gekennzeichnet, dass** die Löcher oder Öffnungen kleiner als die mittlere Faserlänge der Fasern der Vliesmateriallage sind.

16. Windelohr bzw. Windelverschlussband umfassend einen Fasteningträger, der ein Bestandteil einer lösbaren Befestigungseinrichtung in einem Befestigungsbereich trägt und Permanentbereich sowie einen Zwischenbereich aufweist, wobei der Befestigungsbereich und der Permanentbereich nicht-elastisch und der Zwischenbereich elastische ausgebildet ist, gekennzeichet durch einen Fasteningträger aus einem Flächenmaterial nach einem der Ansprüche 9 bis 15.

17. Windel einem Winkelkörper und mit wenigstens einem Windelohr bzw. Windelverschlussband nach Anspruch 16, welches in seinem Permanentbereich permanent mit dem Windelkörper verbunden und in seinem Befestigungsbereich lösbar mit dem Winkelkörper verbindbar ist.

## Claims

1. A method for producing a flat material (11) with elastic and non-elastic regions (12, 13) and also with a textile character at least on one surface side, wherein a fibrous-web material (2, 3) and an elastic film (6) are essentially planarly connected to one another by pressing together and wherein the pressing forces in the elastic regions (12) are lower than in the non-elastic regions (13), **characterised in that** the fibrous-web material (2, 3) is connected in a laminating or connecting unit, which is arranged directly downstream of an extrusion unit for the elastic film (6), to the still plastic film (6) by means of light pressing together, wherein by means of an intensified pressing together in certain regions, the fibrous-web material (2, 3) or the individual fibres thereof are pressed more strongly into the still plastic film (6) than occurs in the remaining regions and/or **in that** the fibrous-web material (2, 3) is supplied in an essentially loose composite of a connecting device (1) before the connection to the elastic film (6) by means of the connecting device (1).

2. The method according to Claim 1, **characterised in that** the arrangement (8) made up of fibrous-web material (2, 3) and elastic film (6) are pressed onto one another by means of a calendering roll (9) with a surface structure, wherein radially protruding regions (10) of the calendering roll (9) preferably form the non-elastic regions (13).

3. The method according to Claim 1 or 2, **characterised in that** the fibrous-web material (2, 3) and the elastic film (6) are connected to one another thermally by means of extrusion lamination or welding.

4. The method according to one of Claims 1 to 3, **characterised in that** the fibrous-web material and the elastic film are glued to one another.

5. The method according to one of Claims 1 to 4, **characterised in that** the fibrous-web material is a non-woven material.

6. The method according to one of Claims 1 to 5, **characterised in that** the fibrous-web material (2, 3) is stabilised by the elastic film (6) during connection to the elastic film (6).

7. The method according to one of Claims 1 to 6, **characterised in that** the elastic film has holes or openings.

8. The method according to Claim 7, **characterised in that** the holes or openings are smaller than the average fibre length of the fibres of the fibrous-web material (2, 3).

9. A flat material (11) with elastic and non-elastic regions (12, 13) and with a textile character at least on one surface side, with a fibrous-web material layer (15, 16) and an elastic film layer (14) which are essentially planarly connected to one another, wherein the connection in the elastic regions (12) is less than in the non-elastic regions (13), **characterised in that** the bonding strength between the fibrous-web fibres and the elastic film layer (14) in the non-elastic regions (13) is at least 1.5 times as large as the bonding strength between the fibrous-web fibres and the elastic film layer (14) in the elastic regions (12).

10. The flat material according to Claim 9, **characterised in that** the fibrous-web layer (15, 16) and the elastic film layer (14) are thermally connected to one another.

11. The flat material according to Claim 9 or 10, **characterised in that** the fibrous-web layer and the elastic film layer are glued to one another.

12. The flat material according to one of Claims 9 to 11, **characterised in that** the fibrous-web layer (15, 16) is a non-woven material.

13. The flat material according to one of Claims 9 to 12, **characterised in that** the bonding strength of the fibrous-web fibres among themselves is at least one order of magnitude lower than the bonding strength between the fibrous-web fibres and the elastic film layer (14).

14. The flat material according to one of Claims 9 to 13, **characterised in that** the elastic film layer has holes or openings.

15. The flat material according to Claim 14, **characterised in that** the holes or openings are smaller than the average fibre length of the fibres of the fibrous-web material layer.

16. A nappy eye or nappy closure strip comprising a fastening support which carries a constituent of a releasable fastening device in a fastening region and has a permanent region and an intermediate region, wherein the fastening region and the permanent region are non-elastic and the intermediate region is elastic, **characterised by** a fastening support made up of a flat material according to one of Claims 9 to 15.

17. A nappy with a nappy body and with at least one nappy eye or nappy closure strip according to Claim 16, which is permanently connected in its permanent region to the nappy body and can be releasably connected to the nappy body in its fastening region.

## Revendications

1. Procédé de fabrication d'une matière de surface avec des zones élastiques et des zones non élastiques (12, 13), ainsi qu'avec un caractère textile (11) au moins sur une face superficielle, alors qu'on relie l'un à l'autre une matière en voile (2, 3) et un film élastique (6) sensiblement à pleine surface par compression l'un sur l'autre, les forces de pression de contact étant plus faibles dans les zones élastiques (12) que dans les zones non élastiques (13), **caractérisé en ce qu'**on relie par légère pression mutuelle la matière en voile (2, 3) dans une unité de contrecollage ou d'assemblage qui est disposée directement derrière une unité d'extrusion pour le film élastique (6) avec le film (6) encore plastique, par une compression mutuelle renforcée dans des zones choisies, la matière en voile (2, 3) ou ses fibres individuelles étant comprimées dans le film (6) encore plastique plus fort que dans les autres zones et/ou **en ce qu'**avant la liaison avec le film élastique (6), la matière en voile (2, 3) est amenée au moyen d'un dispositif d'assemblage (1) dans un composite encore sensiblement lâche du dispositif d'assemblage (1).

2. Procédé selon la revendication 1, **caractérisé en ce qu'**on compresse l'un sur l'autre l'agencement (8) composé de matière en voile (2, 3) et de film élastique (6) via un rouleau de calandre (9) avec une structure superficielle, alors que de préférence des zones (10) saillantes en direction radiale du rouleau de calandre (9) forment les zones non élastiques (13).

3. Procédé selon la revendication 1 ou la revendication 2, **caractérisé en ce qu'**on relie l'un à l'autre la matière non tissée (2, 3) et le film élastique (6) par procédé thermique, par laminage par extrusion ou par soudage.

4. Procédé selon l'une quelconque des revendications 1 à 3, **caractérisé en ce qu'**on colle ensemble la matière en voile et le film élastique.

5. Procédé selon l'une quelconque des revendications 1 à 4, **caractérisé en ce que** la matière en voile est une matière non-tissée.

6. Procédé selon l'une quelconque des revendications 1 à 5, **caractérisée en ce que** lors de l'assemblage avec le film élastique (6), on stabilise la matière en voile (2, 3) par le film élastique (6).

7. Procédé selon l'une quelconque des revendications 1 à 6, **caractérisé en ce que** le film élastique comporte des trous ou des orifices.

8. Procédé selon la revendication 7, **caractérisé en ce que** les trous ou orifices sont plus petits que la longueur moyenne des fibres de la matière en voile (2, 3).

9. Matière de surface (11) avec des zones élastiques et des zones non élastiques (12, 13), ainsi qu'avec un caractère textile au moins sur une face superficielle, avec une couche de matière en voile (15, 16) et une couche de film élastique (14) qui sont reliées l'une à l'autre sensiblement à pleine surface, dans les zones élastiques (12), la liaison étant plus faible que dans les zones non élastiques (13), **caractérisée en ce que** la force de liaison entre les fibres du voile et la couche de film élastique (14) dans les zones non élastiques (13) correspond au moins à 1,5 fois la force de liaison entre les fibres du voile et la couche de film élastique (14) dans les zones élastiques (12).

10. Matière de surface selon la revendication 9, **caractérisée en ce que** la couche de matière en voile (15, 16) et la couche de film élastique (14) sont reliées l'une à l'autre par procédé thermique.

11. Matière de surface selon la revendication 9 ou la revendication 10 **caractérisée en ce que** la couche de matière en voile et la couche de film élastique sont collées l'une sur l'autre.

12. Matière de surface selon l'une quelconque des revendications 9 à 11, **caractérisée en ce que** la couche de matière en voile (15, 16) est une matière non-tissée.

13. Matière de surface selon l'une quelconque des revendications 9 à 12, **caractérisée en ce que** la force de liaison mutuelle entre les fibres du voile se situe au moins dans un ordre de grandeur inférieur à la force de liaison entre les fibres du voile et la couche de film élastique (14).

14. Matière de surface selon l'une quelconque des revendications 9 à 13, **caractérisée en ce que** la couche de film élastique comporte des trous ou des orifices.

15. Matière de surface selon la revendication 14, **caractérisée en ce que** les trous ou les orifices sont plus petits que la longueur moyenne des fibres de la couche de matière en voile.

16. Bord de couche ou bande de fermeture de couche, comprenant un support de fermeture qui est un élément d'un dispositif de fixation amovible dans une zone de fixation et qui comporte une zone permanente, ainsi qu'une zone intermédiaire, la zone de fixation et la zone permanente étant conçues en version non élastique et la zone intermédiaire étant conçue en version élastique, caractérisé(e) par un support de fermeture en une matière de surface selon l'une quelconque des revendications 9 à 15.

17. Couche avec un corps de couche et avec au moins un bord de couche ou une bande de fermeture de couche selon la revendication 16, qui dans sa zone permanente est relié(e) de manière permanente avec le corps de couche et dans sa zone de fixation peut se relier de manière amovible avec le corps de couche.
